# EUROPEAN PATENT APPLICATION

(11) **EP 3 991 767 A1**
(43) Date of publication of application: **04.05.2022**
(21) Application number: 20204338.6
(22) Date of filing: 28.10.2020
(51) Int. Cl.: A61M 1/16, A61M 1/28

(54) **APPARATUS FOR PUMPING AND DIALYSIS**

(71) Applicant: Bellco S.r.l., 41037 Mirandola (MO) (IT)
(72) Inventor: ORLANDINI, Salvatore, 41037 Modena (IT); PETRUCCI, Alberto, 41037 Modena (IT); MARRA, Antonia Giuseppe, 41037 Modena (IT)
(74) Representative: Maschio, Antonio

(57) **Abstract**

In medical apparatus, for example a dialysis machine, a pump is used to move fluid such as purified water or dialysate. The pump performance is monitored by determining the weight or mass of the fluid being pumped. A less expensive pump may be used whilst maintaining an accurate determination of the volume pumped.

## Description

### FIELD OF THE INVENTION

This invention relates to medical apparatus in particular for dialysis and a pumping apparatus for use in such apparatus.

### BACKGROUND

One type of dialysis for the treatment of kidney failure is peritoneal dialysis (PD) which uses the lining of a patient's abdomen, the peritoneum, to filter blood while it remains inside the body. Peritoneal Dialysis is an effective way to treat patients with End Stage Renal Disease and acute patients with renal failure. The patient is provided with a catheter that includes a transfer set for selective connection to a dialysis solution or a drain bag. A dialysis solution ('dialysate' or 'peritoneal dialysis fluid') comprising water with other electrolytes is warmed to body temperature and then introduced into the peritoneal cavity of the patient via the catheter. The solution remains here for a period of time required to clean the blood of waste products, toxins and excess fluid and these are then removed from the body to leave the right amounts of electrolytes and nutrients in the blood. The machine or 'cycler' may repeat the exchange process a number of times (cycles), as required for a particular patient.

Conventionally dialysate consists of purified water, glucose and electrolytes, with the concentration of electrolytes resembling that which occurs naturally in the blood. It is prepared according to an individual patient's needs to help regulate their electrolyte and acid-base balance and remove metabolic waste products. The dialysate components are normally shipped in the form of concentrates, with basic and acidic concentrates being prepared separately. The basic concentrate is usually supplied as a dry concentrate consisting of pure sodium bicarbonate which is made up into a saturated solution and the acidic concentrate is usually provided as a liquid concentrate. If it is provided as a dry concentrate, the entire concentrate must be dissolved before being diluted to form the dialysate. This requires a large volume of liquid and multiple components for providing the peritoneal dialysis fluid, increasing the time required for production of the fluid. Concentrate may also be wasted during the production of the correct peritoneal dialysis fluid and difficulties may be encountered in providing the correct concentration of electrolytes in the fluid for a particular patient.

In a newly developed dialysis machine it has been proposed to provide an architecture in which one part of the machine will be used to prepare the dialysate from purified water (purifying a domestic water supply) and the concentrate and another part to cycle the prepared dialysate through the patient via a catheter into the patent's peritoneum.

It will be appreciated that a suitable location for a water supply for generating the dialysate may not be conveniently located relative to the where the dialysis is to be performed. For example, the patient may prefer to rest on a bed in a bedroom and the nearest source of water may be located in a bathroom some metres distant.

In order to create the dialysate and to deliver it to the patient a pump is required. As will be understood, pumps for medical equipment need to be accurately controlled with high tolerances of manufacture and to be reliable. The volume of fluid pumped over any given time must predictable to ensure accurate control which means that the pumps are subject to stringent manufacturing tolerances. This makes such pumps expensive. Further, in use, a pump's tolerances may change through environmental factors, e.g. heat or cold or by wear from use. Accordingly, presently utilised pumps are expensive and have to be subject to regular maintenance and expensive replacement.

The invention arose in an attempt to mitigate or reduce these issues.

### SUMMARY OF THE INVENTION

According to the invention there is provided apparatus for pumping a fluid comprising a reservoir for, in use, holding a supply of the fluid, a pump coupled to the reservoir to receive fluid therefrom or to pump fluid thereto, a sensor for determining a variation in the amount of the fluid held in the reservoir and a controller to determine from the variation in the amount a value for the amount of fluid pumped by the pump.

In the preferred embodiment, the amount of fluid is determined by determining weight by providing for example a sensor for measuring weight. The amount may be determined by measuring volume and the pump performance measured in terms of the volume pumped or indeed amount of fluid pumped.

In the specific embodiment, the pump pumps the fluid from the reservoir. However, in alternative embodiments, the pump may pump water into the reservoir. The fluid may be water, or dialysate or other fluids.

The weight variation may be determined by use of a piezoelectric sensor supporting the weight of the fluid and the reservoir. In other embodiments, the amount of fluid may be determined by other types of sensors. For example, sensors to determine the volume of fluid held in the reservoir. For example, a photosensor or electrical contact sensor for determining the level of the fluid held.

In preferred embodiments, a further sensor is provided for determining the number of pump cycles performed by the pump. The sensor or indeed both sensors may be provided as a discrete component or as part of an encoder or processor or integrated circuit and or a mems (micromechanical electrical system). In the described embodiment, a rotary pump is used and the sensor determines the number of revolutions of the pump carried out. This allows the volume pumped per revolution or cycle of the pump to be calculated. Other types of pumps may be used for example pumps which use a piston reciprocating within a cylinder. Such pumps are characterised as having a volume per stroke.

The apparatus provides for a calibration to be performed and the volume pumped per cycle may be used for later calculations to determine the volume pumped by reference to the number of cycles of the pump being counted. In preferred arrangements, the calibration will be repeated periodically to up date the value to be used. This is preferred since it will allow for the ageing and other effects on the performance of the pump to be accommodated.

In a proposed apparatus, the pump is provided as part of a cassette which is loaded into the machine along with other disposable components. When the dialysis is complete the cassette is removed by the user and discarded. At the next dialysis session, a new cassette is loaded with a fresh pump which may have different characteristics to the previously used pump due to manufacturing tolerances. For example, a first pump may have a pump stroke which delivers 0.55ml. A second pump may have a pump stroke of 0.56ml. Thus, the invention allows for the amount of fluid delivered by the pump to be determined to ensure that the correct amount is delivered even where the pump is used once or for the first time and a calibration step need not be carried out.

If the calibration result departs beyond a predetermined threshold, then the pump performance may have varied sufficiently to indicate that the pump is faulty or aged to an extent that it requires replacement. When this is determined, an indication may be given by means of audio or visual alert that servicing is required.

In a further aspect of the invention, a dialysis machine is provided including apparatus in accordance with the first aspect. A dialysis machine comprising apparatus for pumping a fluid as claimed in any preceding claim.

### BRIEF DESCRIPTION OF THE FIGURES

A specific embodiment of the invention will now be described with reference to the figures in which:
Figure 1 shows in schematic form a dialysis machine in accordance with an embodiment of the invention in which a preparator module of the machine is located in a patient's bathroom and a cycler module of the machine is located in the patient's bedroom; and
Figure 2 shows a pumping apparatus in accordance with the invention used in the dialysis machine of figure 1.

### DETAILED DESCRIPTION

### Definitions

Unless defined otherwise, all technical and scientific terms used generally have the same meaning as commonly understood by one of ordinary skill in the art.

The articles "a" and "an" are used to refer to one or to over one (i.e., to at least one) of the grammatical object of the article. For example, "an element" means one element or over one element.

The term "comprising" includes, but is not limited to, whatever follows the word "comprising." Use of the term indicates the listed elements are required or mandatory but that other elements are optional and may be present.

The term "consisting of" includes and is limited to whatever follows the phrase "consisting of." The phrase indicates the limited elements are required or mandatory and that no other elements may be present.

The term "consisting essentially of" includes whatever follows the term "consisting essentially of" and additional elements, structures, acts or features that do not affect the basic operation of the apparatus, structure or method described.

The term "dialysate" describes a fluid into or out of which solutes from a fluid to be dialyzed diffuse through a membrane. An initial dialysate used for therapy typically contains electrolytes close in concentration to the physiological concentration of electrolytes found in blood. However, the concentration of the dialysate can change over the course of therapy, and can further be adjusted as desired.

The term "dialysis flow path" refers to a fluid pathway or passageway configured to convey a fluid, such as dialysate and/or blood, wherein said pathway forms at least part of, preferably the whole of, a fluid circuit for peritoneal dialysis, haemodialysis, hemofiltration, hemodiafiltration or ultrafiltration. A dialysis machine may be included within the flow path. A dialyzer may be included in the flow path.

The term "mix" means to combine one or more substance so that the resulting mixture is not easily separated. Mixing, especially evenly spreading, of solute and solvent aids and speeds the process of dissolution. Mixing can be achieved by any method, including spraying, stirring, shaking or otherwise agitating. The term "improved mixing" refers to a situation wherein the components of a mixture are more evenly distributed and not clumped together. Improved mixing may be achieved, for example, by speeding the mixing up. In the context of a solution, improved mixing results in a solution of the correct concentration because all or most of the solutes dissolve, rather than remaining as clumps or aggregated within the solvent.

The present invention relates to an improved automated peritoneal dialysis machine that includes a preparator and cycler for in situ preparation of peritoneal dialysis fluid (PDF) for delivery and drainage from a patient with communications links established therebetween.

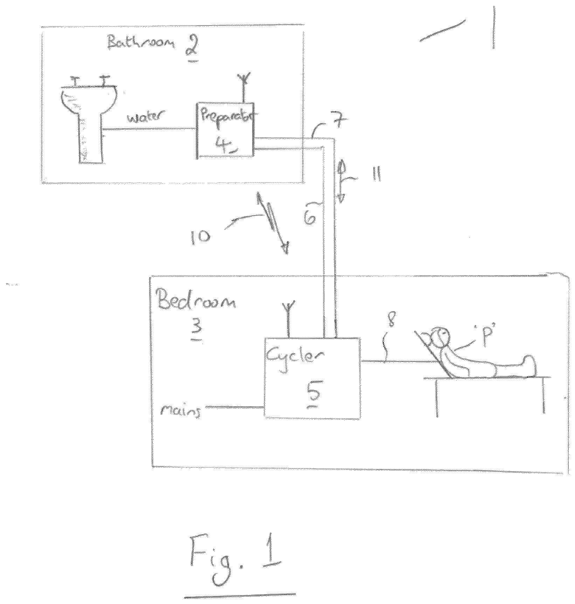

Fig. 1 shows an example of a peritoneal dialysis machine 1 according to the present invention. The peritoneal dialysis machine 1 is set up in the patient's home which is represented by two rooms, a bathroom 2 and an adjoining bedroom 3 (although these rooms are representative only and the locations could be other rooms). The peritoneal dialysis machine 1 includes a preparator 4, an automatic cycler 5, a flexible dialysate supply tube 6 and an electrical power supply line 7.

The preparator 4 is positioned in bathroom 2 as it requires a water source to enable the preparator 4 to generate peritoneal dialysis fluid. The water source may comprise, for example, a tap (faucet) or a more permanent outlet. The preparator 4 first filters the input water and mixes it with a quantity of powered dialysate in accordance with a preprogramed formulation. The powered dialysate will include dextrose, magnesium and calcium and lactate bicarbonate and sodium chloride

The automatic cycler 5 is positioned in the bedroom 3 where the patient "P" intends to undergo peritoneal dialysis. The cycler 5 is connected to a dialysate delivery tube 8 which is connected to a catheter (not shown) which is inserted into the peritoneum of the patient. The cycler 5 controls the flow of the dialysate into, and out of, the patient in accordance with the required dialysis procedure. Dialysate removed from the patient is stored in a container in the cycler 5 (not shown) for subsequent disposal. This procedure is predetermined and held in memory within the cycler 5 operated under processor control. Broadly, the machine operates by preparing dialysate at the preparator 4, the prepared dialysate is pumped via the dialysate supply tube 6 to the cycler 5. There it is heated to patient temperature and administered to the patent via the delivery tube 8 and the catheter into the patient. After, the appropriate time-period for dialysis, the dialysate is removed from the patient via catheter and the delivery tube 8 and held in storage for subsequent removal and disposal.

As will be appreciated, although the two modules are not collocated, it is necessary for instructions and data to pass between the modules. For example, the preparator 4 will need to provide status updates to the cycler 5 indicating that the dialysate is mixed and available for delivery. The cycler 5 will need to instruct the preparator 4 to commence with delivery, or to increase, or reduce the rate of delivery. Error states may need to be passed. For example, the preparator 4 may experience a problem with the water supply pressure and that will need to be communicated to the cycler and indicated as a fault condition on a display. Accordingly, the preparator 4 and the cycler 5 are each provided with a suitably programmed processor to control its respective functions. In addition, a master processor or controller is provided or designated to control the function of the machine as a whole. These processors (or one processor programmed to provide a number of processing functions) provide in addition a communication function to allow the modules to intemperate and to be controlled.

In this embodiment of the invention, there are two communications links which are operable to enable communications between the modules. A first communications link 10 using radio and operating in accordance with a Wi Fi communications protocol and a physical communications link 11 provided by cable 7 over which a Powerlink communications protocol is used.

In order to move the dialysate from the preparator 4 to the cycler 5 and to and from the patient, a pump (or pumps are required). In this case the pump is located at the preparator 4 but it could be located at the cycler or indeed two or more pumps could be provided located at each module.

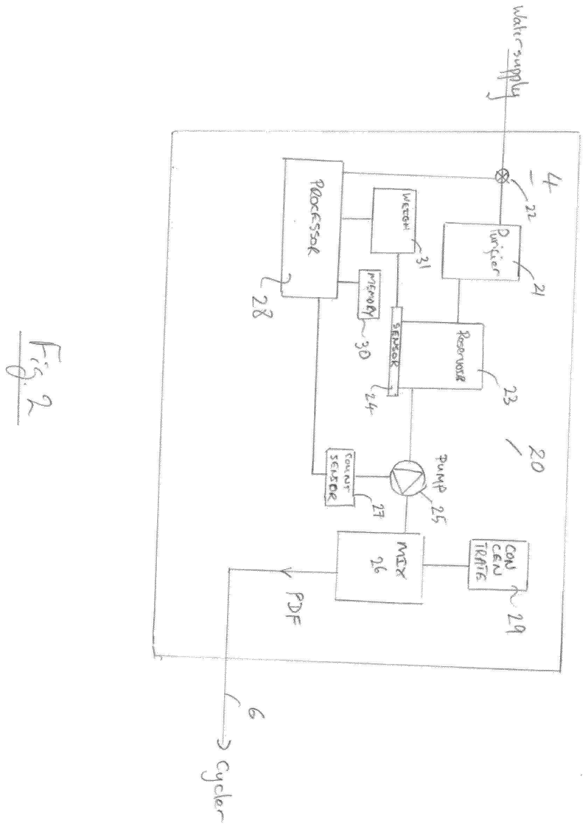

Figure 2 shows a pump apparatus 20 within the preparator 4. It includes a purifier 21 which accepts water via valve 22 from a domestic water supply. The purifier 21 includes filters and a uv light source to purify the mains water and to remove metals or other contaminants. The purifier has an outlet which is coupled to a reservoir 23 where the purified water is stored.

The reservoir is a plastics material container and rests on a piezo electric sensor 24. It has an outlet coupled to a pump 25.

The pump 25 is has an outlet which is coupled to a mixing section 26 and is optically connected to a revolution counting sensor 27. The counting sensor 27 counts the number of pump revolutions and provides the count to a processor 28.

The mixing section 26 accepts dialysate concentrate from a bag 29 and mixes it with the purified water received from the pump 25. The resultant peritoneal dialysis fluid is then output from the preparator 4 to the cycler 5 via the fluid supply line 6.

The processor 28 is a controller controls the functions of the preparator 4 and, in particular, monitors the performance of the pump 25 to determine that the correct amount of fluid is delivered in the following manner. The processor/ controller 28 is a microprocessor operating in accordance with a programme and data held in memory 30.

The sensor 24 provides a signal representative of the weight of the water held in the reservoir 23. This signal is provided to a weight processor 31 which determines the weight and passes this figure to processor 28. The processor 28 determines from the weight at the start of a pumping cycle. At the end of the pumping cycle, the processor determines the current weight of water remaining in the reservoir and determines the difference between that and the initial weight to give a value of the weight of the water delivered to the mixing section. From the weight (mass), the volume of the water may be determined from a predetermine value of water density form the relationship Volume = Mass/ density.

The pump 25 is a rotary pump and the count sensor 27 provides to the processor 28 a value for the number of revolutions of the pump made during the pumping cycle. The volume of the pump stroke, that is to say, the volume of one revolution of the pump, may then be calculated. In essence this will provide a calibration point for the pump. The pump stroke may be used for future calculations of the fluid volume delivered which avoid the necessity to measure the volume using the weight of the water delivered. It may also be recorded over time in a periodic manner, and if it drifts, used as an indication that the pump may need replacement or servicing.

In alternative embodiments, the pump 20 may be located in the cycler with the reservoir holding the PDF and the pumping of that being monitored just prior to delivery to the patient's peritoneum. More than one pump may be provided in each module in alternative embodiments. The pump may be sued to delivery fluid to a reservoir rather than from it and the increase in weight noted to determine the volume of water pumped.

One skilled in the art will understand that various combinations and/or modifications and variations can be made in the described apparatus, methods and uses depending upon the specific needs for operation. Moreover, features illustrated or described as being part of an aspect of the invention may be used in the aspect of the invention, either alone or in combination.

## Claims

**1.** Apparatus for pumping a fluid comprising a reservoir for, in use, holding a supply of the fluid, a pump coupled to the reservoir to receive fluid therefrom or to pump fluid thereto, a sensor for determining a variation in an amount of the fluid held in the reservoir and a controller to determine from the variation an amount of fluid pumped by the pump.

**2.** Apparatus as claimed in claim 1 as claimed in claim 1 wherein the amount is one of volume or weight.

**3.** Apparatus as claimed in claim 1 or 2 further comprising a sensor to provide an output representative of the number of pump cycles performed and to provide the output to the controller which controller being configured to determine therefrom, and the determined amount, a value of the amount of fluid pumped per cycle.

**3.** Apparatus as claimed in claim 2 comprising a memory for storing the determined value of the amount of fluid pumped per cycle of the pump.

**4.** Apparatus as claimed in claim 3 wherein the stored determined value of the volume of fluid pumped per cycle is used by the controller to determine subsequent amounts of fluid pumped by the pump using a count of the cycles performed.

**4.** Apparatus as claimed in claim 3 wherein the controller compares a past determined value of the amount of the fluid pumped per cycle with a more recent determined value of the amount of the fluid pumped per cycle and in the event that the comparison exceeds a threshold performing a recalibration of the pump to determine a current amount of fluid pumped per cycle of the pump and or indicating a pump fault.

**5.** A dialysis machine comprising apparatus for pumping a fluid as claimed in any preceding claim.
